# EUROPEAN PATENT SPECIFICATION

(11) **EP 3 829 615 B1**
(45) Date of publication and mention of the grant of the patent: **14.12.2022**
(21) Application number: 19724271.2
(22) Date of filing: 06.05.2019
(51) Int. Cl.: A61K 36/9066, A61K 9/00, A61K 31/352, A61K 35/644, A61K 36/185, A61K 36/66, A61P 25/00, A61P 9/00, A61P 29/00, A61P 35/00, A61P 37/00, A61P 5/00, A61K 9/20

(54) **COMPOSITION FOR ORAL ADMINISTRATION COMPRISING ONE OR MORE CANNABINOIDS**
ORALE ZUBEREITUNG MIT EINEM ODER MEHREREN CANNABINOIDEN
COMPOSITION POUR UNE ADMINISTRATION ORALE COMPRENANT UN OU PLUSIEURS CANNABINOÏDES

(30) Priority: 08.05.2018 SI 201800089
(43) Date of publication of application: 09.06.2021
(73) Proprietor: Markovic, Danijela, 2000 Maribor (SI); Jovanovic, Sinisa, 2000 Maribor (SI)
(72) Inventor: Markovic, Danijela, 2000 Maribor (SI); Jovanovic, Sinisa, 2000 Maribor (SI)
(74) Representative: Marn, Jure
(86) International application number: PCT/SI2019/050007
(87) International publication number: WO 2019/216832

(56) References cited:
- EP-A2- 1 361 864
- WO-A1-2017/007833
- WO-A1-2017/072704
- CA-A1- 2 993 023
- GB-A- 2 439 393
- US-A1- 2007 104 741
- US-A1- 2016 158 298
- US-A1- 2017 157 041
- A. M. MALFAIT ET AL: "The nonpsychoactive cannabis constituent cannabidiol is an oral anti-arthritic therapeutic in murine collagen-induced arthritis", PROCEEDINGS OF THE NATIONAL ACADEMY OF SCIENCES, vol. 97, no. 17, 15 August 2000 (2000-08-15), pages 9561-9566, XP055202324, ISSN: 0027-8424, DOI: 10.1073/pnas.160105897
- IZZO A A ET AL: "Non-psychotropic plant cannabinoids: new therapeutic opportunities from an ancient herb", TRENDS IN PHARMACOLOGICAL SCIENCES, ELSEVIER, HAYWARTH, GB, vol. 30, no. 10, 1 October 2009 (2009-10-01), pages 515-527, XP026639899, ISSN: 0165-6147, DOI: 10.1016/J.TIPS.2009.07.006 [retrieved on 2009-09-02]

## Description

### Field of technology

The invention relates to the field of pharmaceutical and medical sciences, to the sub-area of natural and complementary medicine and nutritional supplements and supportive therapy.

### Demonstration of the problem solved by the invention

Composition for oral administration comprising one or more cannabinoids, preferably in form of natural orodispersible tablet, according to this invention, solves a technical or health-pharmaceutical problem in the context of the rapid regulation of the endocannabinoid system, which ensures the balanced functioning of all cellular processes and represents a signalling network in the human body. The use of orodispersible tablets will have a quick and optimal effect in the mouth because of its solubility, with rapid and effective absorption or resorption directly through the buccal mucosa.

For purpose of this description, industrial cannabis is a form of cannabis which is a strain of the *Cannabis sativa* plant species that is grown specifically for the industrial uses of its derived products. For purposes of this application industrial cannabis is referred also to as industrial cannabis, or cannabis.

Defective physical and mental balance is a common cause for the emergence of many diseases of modern times. For certain severe forms of illness, with allopathic and homeopathic medical and folk medicine methods, more and more preparations are produced that are not standardized and registered. In most cases, these are products obtained from Indian and industrial cannabis with the addition of chemical substances or ingredients. The problem is, in particular, in unprocessed products of this kind which are not effective, which diminishes the importance of highly cannabinoid substances found in Indian and industrial cannabis.

It is well-known and proven that natural cannabinoids have many healing effects, from relieving symptoms in neurological disorders and stroke, reducing physiological signs of stress, are a strong analgesic with exceptional anti-inflammatory properties, while inhibiting the proliferation of cancer cells and mitigating the side effects of chemotherapy. State of technology or state in pharmacy and health

In the last decade, in developed countries, accelerated research and development in the area of the endocannabinoid system using cannabinoids have been recorded. Cannabinoids, to the full, correspond to endocannabinoid receptors in the human body, which represent one of the physiological super systems for maintaining balance in the body. Endocannabinoid receptors are found in all tissues in the body, they have different intentions and function for the same purpose, that is, the homeostasis of the organism.

After 1988, with the discovery of the first CB1 cannabinoid receptor on the surface of some cells on the body, the mechanism of pain relief became more understandable. In 1993, another type of cannabinoid receptor, CB2, was detected. Because the CB1 receptor is mostly located in the central nervous system, and CB2 only on its periphery and on the cells of the immune system, it is quite understandable and logical that a mixture of natural cannabinoids works against pain and inflammation. This has been demonstrated in the various clinical studies described below.

Among these studies, the most notable one is that of researcher Abrams, who, along with his co-workers, conducted a study in 2009 involving 50 patients with HIV infection, and demonstrated a 30% (w/w) effectiveness in reducing pain and pain sensitivity. Medicines were studied in the combination of THC and CBD natural substances, in different doses.

### Examples of clinical studies

### 1. Br J Clin Pharmacol. 2013 February; 75 (2); 303-12. doi: 10.1111 / j. 1365-2125.2012.04298.x

Study: Cannabidiol as a potential anti-cancer medicine Massi P1, Solinas M, Cinquina V, Paroiaro D.

Several types of evidence support the antitumorigenic effect of cannabinoids, including (9) -tetrahydrocannabinol ((9) -THC), synthetic agonists, endocannabinoids, and endocannabinoid conveyors or degradation inhibitors. Cannabinoids have been shown to have antiproliferative and pro-apoptotic effects known to affect tumour neovascularization, cancer cell transplantation, adhesion, invasion and metastasis. However, the clinical use of (9) -THC and additional cannabinoid agonists is often limited by their undesirable psychoactive effects of THC, and therefore the interest in non-psychoanalytic cannabinoid compounds with structural affinity for (9) -THC, such as cannabidiol (CBD) has significantly increased in the recent years. The study focuses on the efficacy of CBD in modulating different levels of tumorigenicity in several types of cancer, and underlines the importance of investigating THC/CBD analogues as alternative therapeutic agents.

### 2. Br J Pharmaeoi, 2012 Nov; 167 (6): 1218-31. doi: 10.1111 / j. 1476-5381.2012.02050.x

Study: Cannabidiol inhibits angiogenesis by multiple mechanisms.

Solinas M1, Massi P, Canteimo AR, Cattaneo MG, Cammarota R, Bartolini D, Cinquina V, Valenti M, Vicentini LM, Noonan DM, Albini A, Parolaro D.

The study showed antiproliferative and pro-apoptotic actions of cannabinoids on various tumours, together with their anti-angiogenic properties. Non-hypoactive cannabinoid cannabidiol (CBD) effectively inhibits the growth of various types of tumors in vitro and in vivo and reduces certain pro-angiogenic signals produced by glioma cells.

The study also showed that CBD induces HUVEC cytostasis without induction of apoptosis, inhibits HUVEC migration, in vitro invasion and stenography, and in vivo angiogenesis in the sponges of Matrigel. These effects were associated with a reduced modulation of several angiogenesis-associated molecules.

A further study reveals that CBD inhibits angiogenesis with multiple mechanisms. Its double effect on tumor and endothelin cells supports the hypothesis that CBD has potential as an effective agent for the treatment of cancer.

### 3. Endocr Relat Rak. 2008 Jun; 15 (2): 391-408. dol: 10.1677 / ERC-07-0258 Study: Endocannabinoids in endocrine and related tumors. Bifulco M1, Malfitano AM, Pisanti S, Laezza C.

The endocannabinoid system comprises cannabinoid CB 1 and CB2 receptors, their endogenous ligands, endocannabinoids and enzymes that regulate their biosynthesis and degradation. Evidence suggests that endocannabinoids affect intracellular events that regulate the proliferation of many types of endocrine and related cancer cells, leading to an in vitro and in vivo antitumor effect. Endocannabinoids have been shown to inhibit cell growth, invasion and metastasis of the thyroid, chest and prostate tumors.

### 4. FASEB J. 2012 Apr; 26 (4): 1535-48. doi: 10.1096 / fj.11-198184. Epub 2011 December 23

Study: Cannabidiol inhibits lung cancer cell invasion and metastasis via intercellular adhesion molecule-1

Ramer R1, Bublitz K, Freimuth N, Merkord J, Rohde H, Haustein M, Borchert P, Schmuhl E, Linnebacher M, Hinz B.

Cannabinoids inhibit cancer cells invasion via increasing inhibitor of matrix metallo-proteinases-1 (TIMP-1). The study showed that cannabinoids cause ICAM-1, which induces TIMP-1 induction and consequently reduces the invasiveness of cancer cells.

### 5. Sativa for the treatment of multiple sclerosis symptoms Perras C.

Sativex(R) is a cannabis based pharmaceuticalproduct containing 9-tetrahydrocannabinol (THC) and cannabidiol (CBD) in ratio 1:1 and acts as an oral spray. The drug was approved as an additional treatment for neuropathic pain in patients with multiple sclerosis (MS). It is being investigated for treatment of other MS symptoms, such as spasticity. THC: CBD spray is regulated as a narcotic, significantly reducing neuropathic pain, spasticity, muscle spasms and sleep disorders. The most commonly reported adverse reactions (AE) reported in the trials were dizziness, drowsiness, fatigue, feeling of poisoning and poor taste.

### 6, Neurobiol Dis. 2013 nov; 59: 141-50. dor: 10.1016 / j.nbd.2013.06,016. Epub 2013 July 11

Study: Cannabidiol provides long-lasting protection against the deleterious effects of inflammation in a viral model of multiple sclerosis: A role for A2A receptors.

Mecha M1, Feliu A, Inigo PM, Mestre L, Carrillo-Salinas FJ, Guaza C. Inflammation in the central nervous system (CNS) is a complex process involving a plethora of molecules and effectors and requires the migration of blood leukocytes into the blood-brain barrier (HEB) and the activation of resident immune cells. Cannabidiol (CBD) is a nonpsychotropic cannabinoid component of Cannabis sativa, which has strong anti-inflammatory and immunosuppressive properties. The findings highlight the anti-inflammatory effects of CBD in this viral MS model and demonstrate the important therapeutic potential of this compound for the treatment of pathologies with an inflammatory component.

### 7. Curr Med Res opinion. 2007 Jan, 23 (1): 17-24

Study: Meta-analysis of cannabis based treatments for neuropathic and multiple sclerosis-related pain.

Iskedjian M1, Bereza B, Gordon A, Piwko C, Einarson TR.

The pains that arise with 50-70% (w/w) of patients with multiple sclerosis (MS) are poorly understood and rarely studied. Data on the effectiveness and safety of cannabinoid-based medications for neuropathic pain have been summarized.

Cannabinoids, including CBD/THC buccal sprays, have been shown to be effective in treating neuropathic pain in MS.

### 8. Cannabidiol reduces cardiac dysfunction, oxidative stress, fibrosis, and cellular apoptosis in diabetic cardiomyopathy.

Rajesh M1, Mukhopadhyay P, Batkai S, Patel V, Saito K, Matsumoto S, Kashiwaya Y, Horvath B, Mukhopadhyay B, Becker L, Hasko G, Liaudet L, Wink DA, Veves A, Mechoulam R, Pacher P.

Study: To investigate the effects of cannabidiol (CBD) on myocardial dysfunction, inflammation, oxidative/nitrate stress, cell death, and interconnected signaling pathways by using the mouse model of type I diabetic cardiomyopathy and primary human cardiomyocytes exposed to high glucose. Take into account that cannabidiol is the most comprehensive non-psychoactive component of the Cannabis sativa (marijuana) plant that has anti-inflammatory effects in various disease models and relieves pain and spasticity associated with multiple sclerosis in humans. The results have shown that CBD has great therapeutic potential in humans for the treatment of diabetic complications and possibly other cardiovascular disorders by reducing oxidative/nitrate stress, inflammation, cell death, and fibrosis.

### 9. Study: The nonpsychoactivc cannabis constituent cannabidiol is an oral anti-arthritic therapeutic in murine collagen-induced arthritis.

Malfait AM1, Gallily R, Sumariwalla PF, Malik AS, Andreakos E, Mechoulam R, Feldmann M.

The study showed that CBD was able to block an increase in the tumor factor of tumor necrosis in C57 / BL mice that caused lipopolysaccharide. These findings prove that the CBD has a strong anti-arthritic effect in murine collagen-induced arthritis.

### 10. Neurochem. 2004 Apr; 89 (1): 134-41

Study: Neuroprotective effect of cannabidiol, a non-psychoactive component from Cannabis sativa, on beta-amyloid-induced toxicity in PC12 cells.

luvone T1, Esposito G, Esposito R, Santamaria R, Di Rosa M, Izzo AA. Alzheimer's disease is often associated with oxidative stress, partly due to the membrane effect of beta-amyloid peptide aggregates. The results have shown that cannabidiol carries out a combination of neuroprotective, antioxidant and anti-apoptotic effects against the toxicity of beta-amyloid peptides, and that the inhibition of Caspase 3 appearance from its inactive precancer proxaspase 3 with cannabidiol is involved in signaling, the right path for this neuroprotection.

### 11. Curr Neuropharmacol. 2010 sept 8 (3); 243-53. doi: 10.2174 / 157015910792246173

Study: Cannabinoid receptors as a target for the treatment of osteoporosis.

The central nervous system plays an important role in regulating bone metabolism in health and in diseases in which the number of neurotransmitters affected the activities of bone cells through the central relay. Accordingly, the study has shown that endocannabinoids and their receptors are involved in the pathogenesis of osteoporosis associated with increased bone turnover. This study summarizes the in vitro and in vivo findings regarding the impact of cannabinoid ligands on bone metabolism and advocates the exploitation of cannabinoid receptors as targets for anabolic and anti-resorption therapy for the treatment of complex multifaceted bone diseases such as osteoporosis.

### State of the art or known pharmaceutical solutions

WO2017007833 describes healthful supplements.

For example, the supplements can include at least four of the following types of nutrients: an amino acid blend, a coconut extract, a vegetable glycerin extract, an alcohol blend, a cannabinoid blend, a hemp plant extract, a Boswellia serrata extract, a curcuma / turmeric blend, black cumin seed, an Artemisia ludoviciana extract, an Astragalus extract, a fenugreek extract, a mushroom extract blend, or any combination thereof.

According to the patent document Sl 25164, a natural blend of cannabinoids of Indian and industrial cannabis and excipients is known to relieve symptoms in cachexia and to reduce tumor markers in oncological patients. The product is composed of THC and CBD substances, with the addition of two absorption accelerators and is used in the pharmaceutical form of suppositories, capsules and suppositories. The disadvantage of this known solution is, in particular, the use of Indian cannabis with a high THC value. Furthermore, another disadvantage is in the use of ingredients of monosaccharides, fructose and glucose, which is not acceptable for diabetic patients, as sugars stimulate the growth of cancerous cells. The Nobel Prize winner from 1931 Otto Warburg has developed a hypothesis that cancer cells have a different energy metabolism compared to healthy cells, as they faster absorb a greater amount of sugar which they consume. Increased absorption of glucose, at an early stage, is proven to cause increased production of cancer cells. Furthermore, this known solution has this disadvantage that DMSO (dimethylsulfoxide) is used as the carrier of the active substances, which is used in particular as a preservative for organs and as a solvent, and FDA (Food and Drug Administration) prohibits its use for pharmaceutical and medical purposes.

According to patent document US 2017/0157041 A1, an orally-soluble tablet or cannabis capsule is known, comprising one or more active chemical components based on cannabis and containing one or more non-active ingredients and comprising at least one cannabinoid or terpenoid. Its weakness lies in the fact that it is built from chemical active compounds and chemical or artificial excipients. Since it is compressed from several layers and is film-coated, its disadvantage is also in the fact that it breaks down in layers, and its resorption is slow and ineffective. Furthermore, its weakness is that it contains caffeine that blocks the receptor CB1 through the receptor A1, which means that cannabinoids have no place to bind. Another disadvantage is that it contains preservatives such as methyl paraben E-218, methyl propyl paraben E-216 and sodium metabisulphite E-223, but it also contains a sweetener of sodium saccharin E954, since lactose is not suitable for diabetes and cancer patients because it has high glycaemic index and feeds cancer cells.

The previously mentioned clinical studies and the weaknesses or shortcomings of known solutions from available patent documents have shown that their administration through pharmaceutical forms, such as tablets, spark plugs, sprays and capsules, due to the inadequate composition of the active substance, does not give the desired effect. The latter represents an unresolved pharmaceutical and, consequently, a medical problem. Furthermore, the weakness of the known solutions is that they contain a very high percentage of THC and that they do not contain absorption accelerators, except in the case of Patent Sl 25164, which is very important in patients with cachexia.

We are not aware of any industrially prepared drug from a mixture of natural cannabinoids, as well as products prepared exclusively from industrial cannabis.

### Description of the invention

The aforementioned pharmaceutical - medical problem will be resolved with a composition for oral administration comprising one or more cannabinoids, preferably in form of natural orodispersible tablet as prepared according to the invention, compriseing of natural active cannabinoids, natural additional active ingredients and natural excipients. Its base is therefore formed of compositions with a CBD content and a minimum THC content, other cannabinoids and other natural active ingredients that enable a rapid intra buccal application to achieve a direct transport of the active substance into the blood. This avoids the effect of the loss of the active substance in the metabolic process, or the functioning of digestive enzymes and gastric acid. In this way, it will be possible to transfer the use of these tablets from the inpatient and outpatient environment to a wider public space, which will be accessible to all who need such treatment. At the same time, it will be possible to guarantee a preventive use of a composition for oral administration comprising one or more cannabinoids, preferably in form of natural orodispersible tablet, which will reduce the possibility of developing various conditions of the disease.

The embodiment of this invention is particular in the form of a natural orodispersible tablet, or more specifically a natural orodispersible tablet of compositions containing CBD and THC and other natural cannabinoids and natural active ingredients which is rapidly dissolved in the mouth and is absorbed intravenously by a rapid mucoadhesive buccal application through the buccal mucosa. It is preferably oval with an evenly distributed active mixture and is directly compressed. It is intended especially for the relief and treatment of symptoms in immunological, carcinogenic, neurological, inflammatory and cardiovascular diseases. It can also be used for other diseases and as a support for the treatment of endocrine disorders.

For purposes of this description, orodispersible tablet has the same meaning as orally dispersing tablet, for example in EP2482822.

In order to provide for appropriate content of CBD, CBD in crystalline form (or in other form suitable for mixing, and increasing total content of CBD) is provided, in this particular case in 99% pure crystalline form.

The embodiment of this invention is shown in form of three different compositions, comprising about 500 mg as follows (all units mg):

| | 50% form. | 30% form. | 20% form |
|---|---|---|---|
| industrial cannabis resin | 92.6 | 55.65 | 37.04 |
| added CBD, preferably in crystalline | | | |
| form | 373.4 | 225.5 | 188.16 |
| cannabis flour | 15 | 49.8 | 100 |
| honey | 2 | 8.4 | 12.4 |
| poppy | 5 | 38.3 | 40 |
| curcuma | 1 | 5.4 | 7.4 |
| cannabis leaves | 10 | 104.5 | 104.5 |
| cannabis protein | 1 | 12.4 | 10 |
| | 500mg | 499.9mg | 499.5mg |

Cannabis flour is obtained, for example, by grinding cannabis seed.

Said compositions are then incorporated into appropriate formulation for oral administration, for example natural orodispersible tablet, orodispersible tablet or similar wherein at least one additional pharmaccutically acceptable ingredient can be added, such as
- an adhesive agent, wherein the adhesive agent is selected from the group consisting of sucrose aluminum sulfate complex, chitosan and derivatives thereof, polyvinylpyrrolidone, methylcellulose, sodium carboxymethylcellulose, hydroxypropyl cellulose, cross-linked or uncross-linked polyacrylates, cross-linked polyarylates, acidic cross-linked or uncross-linked polyacrylates, polyacrylic acid homopolymers or copolymers, aminoalkyl methacrylate copolymers, methacrylic acid/methylmethacrylate copolymer, alkylacrylate/alkylmethacrylate copolymers, ammoniomethacrylate copolymers, carbomer homopolymers or copolymers, hydrophilic polysaccharide gums, maltodextrins, cross-linked alginate gum gels, polycarboxylated vinyl polymers, pectins, xanthan gums, alginic acid, modified alginic acids, and combinations thereof;
- a disintegrant, which can be selected from the group consisting of crospovidone, sodium starch glycolate, crosslinked carboxymethyl cellulose, low-substituted hydroxylpropylcellulose, mannitol, xylitol, sorbitol, maltol, maltitol, lactose, sucrose, maltose, and combinations thereof;
- an excipient, which can be selected from the group consisting of mannitol, xylitol, sorbitol, maltol, maltitol, lactose, sucrose, maltose, cyclodextrin, and combinations thereof.

In one of the embodiments, the orodispersible tablet comprises
- from 30.00mg to 100.00 mg of industrial cannabis resin, said industrial cannabis resin comprising CBD and/or THC;
- from 150.00 mg to 400 mg of additional CBD, preferably in pure (99%) crystalline form;
- from 10.00 mg to 120 mg of cannabis flour;
- from 0.05 mg to 15 mg of honey, preferably acacia honey and/or chestnut honey, more preferably ecological,
- from 0.05 mg to 50.00 mg of poppy, preferably blue poppy,
- from 0.05 mg to 10.00 mg of curcuma, preferably curcuma roots,
- from 1.00 mg to 120.00 mg of cannabis leaves, preferably ground,
- from 0.05 mg to 15.00 mg of cannabis protein,
and at least one pharmaceutically acceptable ingredient as an adhesive agent, an excipient, or disintegrant.

In above referenced example, honey is preferable in form of acacia or chestnut honey, and poppy is preferable in form of blue poppy.

The composition for oral administration comprising one or more cannabinoids, preferably in form of natural orodispersible tablet is preferably oval-shaped and has the characteristic that it rapidly dissolves in the mouth, thereby allowing rapid mucoadhesive buccal application in the human organism. As previously mentioned, it comprises of compositions which are active ingredients, and excipients such as magnesium stearate and stevia. The composition containing CBD and THC was named Sicanada mix. In another embodiment, the orodispersible tablet may also be of different or irregular shapes.

The active composition is evenly distributed throughout the entire composition for oral administration comprising one or more cannabinoids, preferably in form of natural orodispersible tablet, which is consequently homogeneous. At the final stage of its manufacture, magnesium stearate is added to it, which serves as a coating of the tablet in direct compression.

The previously described structure or composition of a composition for oral administration comprising one or more cannabinoids, preferably in form of natural orodispersible tablet allows that the tablet is evenly and rapidly dissolved in the mouth into very small particles so that its active ingredients can simultaneously and equally efficiently penetrate the mucus directly into the blood. Due to the herbal composition, the composition for oral administration comprising one or more cannabinoids, preferably in form of natural orodispersible tablet is green, with no additional coloring agents, and it can have a transverse cut-off on one end of the face which is intended to merely divide the tablet, rather than dividing it into two equal doses.

The present invention of the composition for oral administration comprising one or more cannabinoids, preferably in form of natural orodispersible tablet refers to a mixture or resin from an extract of any kind of industrial cannabis and composition. The extract is in the final product in oral form as a composition for oral administration comprising one or more cannabinoids, preferably in form of natural orodispersible tablet. In general, the optimal effect of CBD products is achieved if they are consumed by placing them under or above the tongue; they dissolve in a very short time through the mucous mucosa to penetrate the blood.

The substances consumed are absorbed in two ways. What is swallowed is absorbed in the intestine, and what is retained in the mouth is absorbed in the oral mucous membrane. Substances absorbed in the mouth come through the oral mucous membranes directly into the bloodstream. First into the right heart, then into the lungs, and then again into the heart and then all over the body. Thus, these substances avoid the liver, which processes and changes substances. Everything that people eat comes through the so-called portal vein from the gastrointestinal tract, first and foremost in the liver, and only then, altered, processed and also detoxified, into the bloodstream, first from the liver to the right heart and into the lungs, then back to the heart and finally all over the body.

Thus, the passage through the oral mucous membrane can be an alternative way of administration of medications in the body. It has its advantages both before and after intravenous administration. It is particularly important for medicines that must function immediately in case when survival depends on the speed of action of the drug, for example in cardiac patients and allergies.

Natural medicinal substances, which are retained for a while under the tongue, are absorbed completely unchanged in a very short time. The oral mucous membrane is strongly circulated and is covered with thick and permeable mucous layer of the mucous membrane and therefore is permeable. The thinnest and most permeable part is below the tongue, followed by the buccal mucous membrane (cheeks and gum), and the least permeable is where it is the thickest, that is, the mucous membrane of the tongue. Absorption in the mouth is via cells or through intercellular spaces. Fat soluble substances pass through the cells, and soluble substances in the water via the intracellular substance.

The area under the tongue is also an energy point that is associated with the adrenal gland. Since both techniques include the functioning of the substance and the functioning of the information, both techniques have a great effect on health and well-being.

The latter was a guide in the development of the composition and shape of a composition for oral administration comprising one or more cannabinoids, preferably in form of natural orodispersible tablet representing a product from a herbaceous mixture with a CBD content and a minimum THC content, whereby the proportion of CBD and THC in the mixture can be adjusted according to the target group and the particular type of disease.

The preparation in the form of a composition for oral administration comprising one or more cannabinoids, preferably in form of natural orodispersible tablet is useful for pharmaceutical or medical purposes, for relief and in some cases for help in the treatment of:
- immunological diseases,
- carcinogenic diseases,
- as support for endocrine disorders,
- neurological diseases,
- inflammatory diseases,
- cardiovascular diseases and some other diseases.

The process of obtaining composition with the content of CBD, THC and other natural cannabinoids for the industrial production of composition for oral administration comprising one or more cannabinoids, preferably in form of natural orodispersible tablets according to the invention essentially comprises of:
- extraction of CBD, THC and other cannabinoids,
- extraction and mixing of herbal bio mixtures and
- addition of excipients.

The composition for oral administration comprising one or more cannabinoids, preferably in form of natural orodispersible tablet which is the subject of the invention is an active ingredient from a composition with a CBD and THC content and other natural cannabinoids and natural active ingredients. The composition of one orodispersible tablet comprises CBD and THC in the form of a resin of industrial cannabis.

The total resin mass of industrial cannabis contains from 10% (w/w) to 50% (w/w) of the CBD and from 0.2% (w/w) to 9.0% (w/w) THC.

If not enough CBD is comprised within said cannabis resin, CBD in crystalline form may be added in order to achieve desired composition.

A composition for oral administration comprising one or more cannabinoids, preferably in form of natural orodispersible tablet which is the subject of the invention is comprised of the following ingredients:
- from 10% (w/w) to 65% (w/w) of industrial cannabis resin,
- from 5% (w/w) to 25% (w/w) of cannabis flour,
- from 1% (w/w) to 8% (w/w) of honey, preferably acacia honey and/or chestnut honey, more preferably ecological,
- from 4% (w/w) to 20% (w/w) of poppy, preferably blue poppy (*papaver somniferum*).
- from 1% (w/w) to 5% (w/w) of curcuma, preferably root of curcuma,
- from 11% (w/w) to 53% (w/w) of cannabis leaves, preferably finely ground, and
- from 1% (w/w) to 8% (w/w) of cannabis protein.

More preferably, composition according to the invention is comprised of
- from 50% (w/w) to 55% (w/w) of industrial cannabis resin,
- from 9% (w/w) to 12% (w/w) of cannabis flour,
- from 2% (w/w) to 3% (w/w) ) of honey, preferably acacia honey and/or chestnut honey, more preferably ecological,
- from 7% (w/w) to 9% (w/w) of poppy, preferably blue poppy (*papaver somniferum*).
- from 1% (w/w) to 2% (w/w) of curcuma, preferably root of curcuma,
- from 15% (w/w) to 29% (w/w) of cannabis leaves, preferably finely ground, and
- from 2% (w/w) to 3% (w/w) of cannabis protein.

Even more preferably, composition according to the invention is comprised of
- about 52,5% (w/w) of industrial cannabis resin,
- about 10,5% (w/w) of cannabis flour,
- about 2,6% (w/w) of honey, preferably acacia honey and/or chestnut honey, more preferably ecological;
- about 8,1% (w/w) of poppy, preferably blue poppy (*papaver somniferum*).
- about 1,6% (w/w) of curcuma, preferably root of curcuma,
- about 22,1% (w/w) of cannabis leaves, preferably finely ground, and
- about 2,6% (w/w) of cannabis protein.

To the composition, an active carrier of the active substance colloidal silicon dioxide of 0.8% (w/w) to 3% (w/w) is added.

Optionally, about 40% (w/w) to about 60% (w/w) of the composition further comprises from about 0.8% (w/w) to about 3% (w/w) of active carrier of the active substance.

Method for oral administration of a cannabinoid composition, the method comprising administering to a subject composition comprising
an active ingredient in form of an industrial cannabis resin comprising CBD and THC;
said industrial cannabis resin comprising from about 10% (w/w) to about 50% (w/w) of CBD and from about 0.2% (w/w) to about 9.0% (w/w) THC;
wherein said composition is comprised of
   - from 10% (w/w) to 65% (w/w) of industrial cannabis resin,
   - from 5% (w/w) to 25% (w/w) of cannabis flour,
   - from 1% (w/w) to 8% (w/w) of honey, preferably acacia honey and/or chestnut honey, more preferably ecological,
   - from 4% (w/w) to 20% (w/w) of poppy, preferably blue poppy (*papaver somniferum*).
   - from 1% (w/w) to 5% (w/w) of curcuma, preferably root of curcuma,
   - from 11% (w/w) to 53% (w/w) of cannabis leaves, preferably finely ground, and
   - from 1% (w/w) to 8% (w/w) of cannabis protein.

The volume or mass percentages or proportions represent the amount of individual ingredients within the total weight of the active compound. Their value and their interrelation depends on the type of illness that is being treated and its intensity, and from the patient's condition and the current quality of his vital functions.

In order to provide for sufficient quantity of CBD in a composition, from 15% (w/w) to 40,5% (w/w), preferably in pure or almost pure (99%) crystalline form.

An orodispersible tablet comprising from 30% (w/w) to 60% (w/w) of said composition as described above, and at least one pharmaceutically acceptable ingredient as an adhesive agent, an excipient, or disintegrant

As previously described, a plant magnesium stearate is added in the final stage of the tablet's production, with which the active composition for oral administration comprising one or more cannabinoids, preferably in form of natural orodispersible tablet is coated.

Because the mixture of composition for oral administration comprising one or more cannabinoids, preferably in form of natural orodispersible tablet is homogenously mixed and directly compressed, its resorption or absorption is rapid and effective, and is carried out through the buccal mucosa. Because of the faster resorption, the bioavailability of the active substance is higher, as it is not affected by digestive enzymes and gastric acid.

The recommended dose is one to two natural orodispersible tablets per day, also more; depending on the type of illness and body weight of the patient.

### The advantage of the invention over known solutions

For this purpose, there exist only products in the form of orally soluble tablets from one or more chemically active and one or more chemically inactive constituents, in the form of suspensions, suppositories, oromucosal sprays and in the form of a capsule. Wc arc not aware of any such a product in the form of a natural orodispersible tablet that would be entirely made from natural substances, a composition and added CBD and THC, and the rest of the added natural cannabinoids and natural active ingredients.

Composition for oral administration comprising one or more cannabinoids, preferably in form of natural orodispersible tablet represents a new pharmaceutical product in the field of medicine which improves the absorption rate of the CBD itself. In contrast to the already known solutions containing a very high percentage of THC, the natural orodispersible tablet has a low percentage of THC and a possibility of variation of the CBD concentration from 20% (w/w) to 50% (w/w) per tablet. As previously mentioned, the main disadvantage of all known pharmaceutical forms is in the fact that they do not contain absorption promoters or re-absorption, except in the case of a Patent Sl 25164, which is very important in patients with cachexia. A colloid silicon dioxide is used as a transporter in the natural orodispersible tablet, unlike in other tablets.

### Process for obtaining the composition

Below, process for obtaining the composition according to this invention is described in detail.

### Obtaining CBD and Other Cannabinoids:

1. 10 kg of cleaned tips of industrial cannabis (Kannabis Sativa L.) are exposed to the UW light bulb for 24 hours, as this reduces the concentration of THC.
2. The irradiated mixture is frozen for 24 hours at a temperature of -25 to -35 degrees. All ethanol is at this temperature all the time. After 24 hours, pour the mixture with 50 liters of 95% (w/w) ethanol and mix with a stirrer. Leave to stand for 1-3 hours and filter.
3. The residue of the pre-filtered homogeneous mixture is placed under a press. The extracted extract is also filtered out.
4. The resulting residue is returned to the container, which is again cooled and then poured with the same amount of ethanol. This procedure can be repeated 3-5 times. Leave the extract left until the entire procedure is completed.
5. The obtained extract is filtered (at least 3x).
6. Pre-filtered extract is evaporated on a water bath at 60-75 degrees until all ethanol is evaporated.
7. The homogenous mixture obtained is placed in the oven at a temperature of 70-100 degrees for 20-40 minutes. This achieves dexroxilation. Thus, the homogeneous mixture (resin) is prepared for further processing.
8. The obtained homogenous extract is passed through the lyophilization process.

Approximately 200 g of resins are obtained from 10 kg of dry industrial cannabis after such a process.

Composition according to this specification can be in the form comprising a tablet, preferably orodispersible tablet, a capsule, a caplet, a troche, a lozenge, an oral powder, a solution, a thin strip, an oral thin film (OTF), an oral strip, a syrup, a suspension ,or an inhalation compound.

### Extraction and mixing of herbal bio mixtures:

1. A homogeneous mixture (resin) of the cannabis resin is added at a temperature of about 40 degrees to honey (preferably acacia ecological honey).
2. Raising cannabis leaves and obtaining finely ground cannabis into which the preformed mixture is mixed.
3. Harvesting the remains of pressed cannabis seeds and get cannabis flour.
4. The whole cannabis of the seeds, which is separated from peeling, peel and sift, in order to obtain cannabis proteins.
5. Grinding of the blue poppy.
6. All the ingredients are sieved to obtain particles of essentially the same size.
7. All the prepared ingredients are placed in a vertical mixer, where curcuma (*curcuma longa*) is added at a moderate rate of stirring.
8. So is obtained a bio herb blend, which is for purposes of this specifications named Sicanada mix.
9. Repeat the process of lyophilization to the so obtained bio composition.

By the lyophilization process, the adhesiveness of the composition is reduced to facilitate the passage through the capillary tablet charger. Adhesiveness is caused by polar aqueous molecules having covalent bonds and, due to polarity, causing adhesion to the hydrogen bond.

The lyophilization aims to remove moisture - water from the basic matrix without damaging the bioactive substance (drying therefore fails because it is thermally destroyed). When the amount of water in the matrix decreases, adhesion decreases due to the lower potential of hydrogen bonds.

The so prepared mixture (Sicanada mix) is shed due to the flowability and uniform particle size of the powder. When this is achieved, the mixture is prepared for addition, mixing and homogenization with excipients.

### Addition of excipients:

In the vertical mixer, first put the prepared mixture of Sicanada mix and then gradually add pre-screened excipients. First, xylitol, then colloidal silicon dioxide, stevia, and finally magnesium stearate are added.

The Sicanada mix according to one of the embodiments is comprised of:
- resin industrial cannabis (with its cannabinoids supports our endocannobinoid system and regulates the homeostasis of the organism).
- cannabis flour (a rich source of proteins, minerals and fiber).
- acacia or chestnut honey (has a low glycemic index and with its chemical structure accounts for 70% (w/w) of blood plasma, any honey with low glycemic index can be used instead).
- blue poppy (beneficial effect on the cardiovascular system and as a preventive and curative in cancerous prostate and breast disorders).
- turmeric (is an antioxidant and beneficial for all cancers).
- industrial cannabis finely ground (it works calmly, strengthens the immune system and has a beneficial effect on the blood vessels, heart and digestion. It helps to lower cholesterol and sugar in the blood, contributes to a better overall well-being and is an excellent antioxidant).
- cannabis protein (rich in omega acids).

| | |
|---|---|
| Resin industrial cannabis | 6.749,30 kg |
| Cannabis flour | 1.349,86 kg |
| Acacia honey | 0.337,47 kg |
| Blue poppy | 1.038,71 kg |
| Kurkuma | 0.202,48 kg |
| Industrial cannabis finely ground | 2.834,71 kg |
| Cannabis protein | 0.337,47 kg |
| Total | 12.850,00 kg |

Example 1 on the batch:

| | |
|---|---|
| Sicanada mix | 12,850 kg |
| Xylitol | 10,280 kg |
| Silicon dioxide colloidal | 0,257 kg |
| Stevia | 0,025 kg |
| Magnesium stearate | 0,385 kg |
| | 23,797 kg |

Example 2 at tablets masses 926 mg:

| | |
|---|---|
| Sicanada mix | 500 mg |
| Xylitol | 400 mg |
| Silicon dioxide colloidal | 10 mg |
| stevia | 1 mg |
| Magnesium stearate | 15 mg |
| | 926 mg |

Of the total weight of the above example, 24.737,00 tablets containing 50% (w/w) CBD are obtained.

The recommended dose is 1-2 tablets a day, depending on the patient's problems that are resolved and the body weight of the patient.

### Recommended CBD content by type of disease:

- IMMUNOLOGY 1 to 2 tablets a day (with a content of 10 to 30% (w/w) CBD)
- CARCINOGEN 1-2 tablets per day (with a content of 40 to 50% (w/w) CBD)
- support for ENDOCRINED DISEASES 1 tablet per day (with a content of 15 to 20% (w/w) CBD)
- NEUROLOGIC 1-2 tablets per day (with a content of 30 to 50% (w/w) CBD)
- CARDIOVASCULAR 1 tablet per day (with a 30% (w/w) CBD content).

### Studies

There were several studies performed in order to test effectiveness of the composition.

### 1. Male 41 years old

Diagnosis: major lesions on sele turcika
- body spasms;
- caved in spinal channel;
- chronic pain syndrome;
- hypertension, not stable despite therapy

Treatment with composition comprising 50% (w/w) CBD and 0,2% (w/w) THC 2x a day
After 1 months of regular therapy, hypertension is stable
After 5 months of therapy, spasms almost disappear
and pains are occasional

### 2. Male 53 years old

Diagnosis: Low-threshold AP
Hyperlipidemia

When taking a composition of 30% (w/w) CBD and 0.2% (w/w) THC 1 × daily, lowering and stability of blood fat level

| | Previously | after administration |
|---|---|---|
| Cholesterol | 5.17 | 4.33 |
| Triglycerides | 5.44 | 1.52 |
| HDL | 0.87 | 1.14 |
| LDL | 2.16 | 2.50 |

### 3. 10 women with different types of breast cancer

In all cases, the tumor has been surgically removed. Four have received chemo and irradiation after surgery within 3 months. Complications of tumor recurrence on the same breast or on the other, lung lesions and endometrial complications. Following administration of composition with 50% (w/w) CBD and 0.2 % (w/w) THC 2 × per day, the complication is eliminated and the medical condition stabilized. Six did not receive chemo and irradiation after surgery. Following same treatment as the four before, no recurrence of tumor on the breast and without other complications.

### 4. Male 40 years old

Diagnosis: GERB i.o.
Lymphadentitis

Administration of composition comprising 30% (w/w) CBD and 0.2% (w/w) THC 1 × per day

In 2 months the problems disappear.

### 5. Male 65 years old

Diagnosis: low-grade follicular lymphoma
Stage IV
Numerous enlarged lymph nodes.

Administration of composition with 50% (w/w) CBD and 9% (w/w) THC 2 × per day. After 6 months of therapy, increased lymph nodes disappear, they do not expand, no further detected problems, appetite appropriate, physical activity appropriate.

### 6. Male 78 years old

Diagnosis: COPD
Round lesion at the thoracic wall 16 × 14 mm

Administration of composition comprising 50% (w/w) CBD and 0.2% (w/w) THC 2 × per day

After 4 months of therapy, CT scan, pathological lesions or other pathological concentrations in lungs do not appear. The tumor formation is no longer seen. Pulmonary function:

| | Before therapy | after therapy |
|---|---|---|
| VC | 2.8(84%) | 2.6(81%) |
| FV | 1.24 (50%) | 1.25 (50%) |
| Tiff | 43% | 73% |

Therapy continues 1 × per day comprising 50% (w/w) CBD and 0.2% (w/w) THC

### 7. Male 78 years old

Diagnosis: Male liver i.o.
Bastards on the bones

Administration of composition comprising 50% (w/w) CBD and 0.2% (w/w) THC 2 × per day

After 4 months, CT does not show more lesions (previously measuring 7mm), nor any pathological changes on the skeleton. Tumor markers within normal limits.

Continuing with therapy 1 × per day

### 8. Male 56 years old

Diagnosis: lung sarcoidosis and lymph nodes
Sarkoidosis of organs
Regular therapy: Medrol 32mg and 16mg
Altered
Methotrexate 4 tab. per day

Regular therapy was gradually changed with administration of composition with 50% (w/w) CBD and 0.2% (w/w) THC 1 × per day. After 8 months of therapy, the problems decrease, condition is stable
Lung function:

| | Before therapy | after therapy |
|---|---|---|
| FEVI | 2.911 | 3.24 |
| VC | 4.251 (86%) | 4.671 (86%) |
| VI | 2.91 (77%) | 3.2 (86%) |
| Tiff | 68% | 68.5% |
| KCO | 78% | 92% |
| VA | 73% | 86.9% |

## Claims

1. A composition for oral administration comprising one or more cannabinoids, preferably in form of natural orodispersible tablet, comprising an active ingredient in form of an industrial cannabis resin comprising CBD and THC;
wherein said industrial cannabis resin comprises from about 10% (w/w) to about 50% (w/w) of CBD and from about 0.2% (w/w) to about 9.0% (w/w) THC wherein said composition is comprised of
- from 10% (w/w) to 65% (w/w) of said industrial cannabis resin,
- from 5% (w/w) to 25% (w/w) of cannabis flour,
- from 1% (w/w) to 8% (w/w) of honey, preferably acacia honey and/or chestnut honey, more preferably ecological,
- from 4% (w/w) to 20% (w/w) of poppy, preferably blue poppy (papaver somniferum).
- from 1% (w/w) to 5% (w/w) of curcuma, preferably root of curcuma,
- from 11% (w/w) to 53% (w/w) of cannabis leaves, preferably finely ground, and
- from 1% (w/w) to 8% (w/w) of cannabis protein.

2. The composition according to claim 1 wherein said composition is comprised of
- from 50% (w/w) to 55% (w/w) of industrial cannabis resin,
- from 9% (w/w) to 12% (w/w) of cannabis flour,
- from 2% (w/w) to 3% (w/w) ) of honey, preferably acacia honey and/or chestnut honey, more preferably ecological,
- from 7% (w/w) to 9% (w/w) of poppy, preferably blue poppy (papaver somniferum).
- from 1% (w/w) to 2% (w/w) of curcuma, preferably root of curcuma,
- from 15% (w/w) to 29% (w/w) of cannabis leaves, preferably finely ground, and
- from 2% (w/w) to 3% (w/w) of cannabis protein.

3. The composition according to any of previous claims wherein said composition is comprised of
- about 52,5% (w/w) of industrial cannabis resin,
- about 10,5% (w/w) of cannabis flour,
- about 2,6% (w/w) of honey, preferably acacia honey and/or chestnut honey, more preferably ecological;
- about 8,1% (w/w) of poppy, preferably blue poppy (*papaver somniferum*).
- about 1,6% (w/w) of curcuma, preferably root of curcuma,
- about 22,1% (w/w) of cannabis leaves, preferably finely ground, and
- about 2,6% (w/w) of cannabis protein.

4. The composition according to any of previous claims wherein about 40% (w/w) to about 60% (w/w) of the composition further comprises from about 0.8% (w/w) to about 3% (w/w) of active carrier of the active substance.

5. The composition according to claim 4 wherein said active substance is colloidal silicon dioxide.

6. The composition according to any of preceding claims wherein said composition for oral administration is in the form of natural orodispersible tablet, said natural orodispersible tablet consisting of a composition, added CBD and THC, and added natural cannabinoids and natural active ingredients.

7. The composition according to any of preceding claims wherein said natural orodispersible tablet can be of any regular or irregular compressed shape, and further, coated with a magnesium stearate coating.

8. The composition according to any of preceding claims wherein said composition comprises a combination of CBD and THC, or an isoform, a derivative, a precursor, or a metabolite thereof.

9. The composition according to any of preceding claims for use in treatment or prevention of diseases or disorders from the group consisting of immunological diseases, carcinogenic diseases, endocrine disorders, neurological diseases, inflammatory diseases, cardiovascular diseases, or combination thereof.

10. The composition according to any of claims 1 to 8 or the composition for use according to claim 9, wherein the composition is in the form comprising a tablet, a capsule, a caplet, a troche, a lozenge, an oral powder, a solution, a thin strip, an oral thin film (OTF), an oral strip, a syrup, a suspension ,or an inhalation compound.

11. Cannabinoid composition comprising one or more cannabinoids, preferably in form of natural orodispersible tablet, comprising an active ingredient in form of an industrial cannabis resin comprising CBD and THC;
wherein said industrial cannabis resin comprises from about 10% (w/w) to about 50% (w/w) of CBD and from about 0.2% (w/w) to about 9.0% (w/w) THC; wherein said composition is comprised of
- from 10% (w/w) to 65% (w/w) of said industrial cannabis resin,
- from 5% (w/w) to 25% (w/w) of cannabis flour,
- from 1% (w/w) to 8% (w/w) of honey, preferably acacia honey and/or chestnut honey, more preferably ecological,
- from 4% (w/w) to 20% (w/w) of poppy, preferably blue poppy (*papaver somniferum*).
- from 1% (w/w) to 5% (w/w) of curcuma, preferably root of curcuma,
- from 11% (w/w) to 53% (w/w) of cannabis leaves, preferably finely ground, and
- from 1% (w/w) to 8% (w/w) of cannabis protein.

12. The composition according to any of preceding claims further comprising 15% (w/w) to 40,5% (w/w) of CBD, preferably in pure (99% w/w) crystalline form.

13. An orodispersible tablet comprising from 30% (w/w) to 60% (w/w) of said composition in accordance with any of the claims 1 to 12, and at least one pharmaceutically acceptable ingredient as an adhesive agent, an excipient, or disintegrant

14. An orodispersible tablet, comprising,
- from 30.00 mg to 100.00 mg of industrial cannabis resin, said industrial cannabis resin comprising CBD and/or THC, wherein said industrial cannabis resin comprises from about 10% (w/w) to about 50% (w/w) of CBD and from about 0.2% (w/w) to about 9.0% (w/w) THC;
- from 150.00 mg to 400 mg of additional CBD, preferably in pure (99%) crystalline form;
- from 10.00 mg to 120 mg of cannabis flour;
- from 0.05 mg to 15 mg of honey, preferably acacia honey and/or chestnut honey, more preferably ecological,
- from 0.05 mg to 50.00 mg of poppy, preferably blue poppy,
- from 0.05 mg to 10.00 mg of curcuma, preferably curcuma roots,
- from 1.00 mg to 120.00 mg of cannabis leaves, preferably ground,
- from 0.05 mg to 15.00 mg of cannabis protein,
and at least one pharmaceutically acceptable ingredient as an adhesive agent, an excipient, or disintegrant.

15. The composition according to any of claims 1 to 8 or 11 to 12, or orodispersible tablet according to any of claims 13 to 14 for use in treatment or prevention of diseases or disorders from the group consisting of immunological diseases, carcinogenic diseases, endocrine disorders, neurological diseases, inflammatory diseases, cardiovascular diseases, or combination thereof.

## Patentansprüche

1. Zusammensetzung zur oralen Verabreichung, umfassend ein oder mehrere Cannabinoide, vorzugsweise in Form einer natürlichen Schmelztablette, die einen Wirkstoff in Form eines industriellen Cannabisharzes umfasst, das CBD und THC umfasst;
wobei das industrielle Cannabisharz von etwa 10 % (Gew./Gew.) bis etwa 50 % (Gew./Gew.) CBD und von etwa 0,2 % (Gew./Gew.) bis etwa 9,0 % (Gew./Gew.) THC umfasst, wobei die Zusammensetzung besteht aus
- von 10 % (Gew./Gew.) bis 65 % (Gew./Gew.) des industriellen Cannabisharzes,
- von 5 % (Gew./Gew.) bis 25 % (Gew./Gew.) Cannabismehl,
- von 1 % (Gew./Gew.) bis 8 % (Gew./Gew.) Honig, vorzugsweise Akazienhonig und/oder Kastanienhonig, besonders bevorzugt ökologisch,
- von 4 % (Gew./Gew.) bis 20 % (Gew./Gew.) Mohn, vorzugsweise Blaumohn (Papaver somniferum),
- von 1 % (Gew./Gew.) bis 5 % (Gew./Gew.) Kurkuma, vorzugsweise Kurkumawurzel,
- von 11 % (Gew./Gew.) bis 53 % (Gew./Gew.) Cannabisblätter, vorzugsweise fein gemahlen, und
- von 1 % (Gew./Gew.) bis 8 % (Gew./Gew.) Cannabisprotein.

2. Zusammensetzung nach Anspruch 1, wobei die Zusammensetzung besteht aus
- von 50 % (Gew./Gew.) bis 55 % (Gew./Gew.) industriellem Cannabisharz,
- von 9 % (Gew./Gew.) bis 12 % (Gew./Gew.) Cannabismehl,
- von 2 % (Gew./Gew.) bis 3 % (Gew./Gew.) ) Honig, vorzugsweise Akazienhonig und/oder Kastanienhonig, besonders bevorzugt ökologisch,
- von 7 % (Gew./Gew.) bis 9 % (Gew./Gew.) Mohn, vorzugsweise Blaumohn (Papaver somniferum),
- von 1 % (Gew./Gew.) bis 2 % (Gew./Gew.) Kurkuma, vorzugsweise Kurkumawurzel,
- von 15 % (Gew./Gew.) bis 29 % (Gew./Gew.) Cannabisblätter, vorzugsweise fein gemahlen, und
- von 2 % (Gew./Gew.) bis 3 % (Gew./Gew.) Cannabisprotein.

3. Zusammensetzung nach einem der vorhergehenden Ansprüche, wobei die Zusammensetzung besteht aus
- etwa 52,5 % (Gew./Gew.) industriellem Cannabisharz,
- etwa 10,5 % (Gew./Gew.) Cannabismehl,
- etwa 2,6 % (Gew./Gew.) Honig, vorzugsweise Akazienhonig und/oder Kastanienhonig, besonders bevorzugt ökologisch;
- etwa 8,1 % (Gew./Gew.) Mohn, vorzugsweise Blaumohn (*Papaver somniferum*),
- etwa 1,6 % (Gew./Gew.) Kurkuma, vorzugsweise Kurkumawurzel,
- etwa 22,1 % (Gew./Gew.) Cannabisblätter, vorzugsweise fein gemahlen, und
- etwa 2,6 % (Gew./Gew.) Cannabisprotein.

4. Zusammensetzung nach einem der vorhergehenden Ansprüche, wobei etwa 40 % (Gew./Gew.) bis etwa 60 % (Gew./Gew.) der Zusammensetzung ferner von etwa 0,8 % (Gew./Gew.) bis etwa 3 % (Gew./Gew.) eines aktiven Trägers der Wirksubstanz umfasst.

5. Zusammensetzung nach Anspruch 4, wobei die Wirksubstanz kolloidales Siliciumdioxid ist.

6. Zusammensetzung nach einem der vorhergehenden Ansprüche, wobei die Zusammensetzung zur oralen Verabreichung in Form einer natürlichen Schmelztablette vorliegt, wobei die natürliche Schmelztablette aus einer Zusammensetzung, zugesetztem CBD und THC und zugesetzten natürlichen Cannabinoiden und natürlichen Wirkstoffen besteht.

7. Zusammensetzung nach einem der vorhergehenden Ansprüche, wobei die natürliche Schmelztablette jede regelmäßige oder unregelmäßige komprimierte Form haben und ferner mit einer Magnesiumstearatbeschichtung beschichtet sein kann.

8. Zusammensetzung nach einem der vorhergehenden Ansprüche, wobei die Zusammensetzung eine Kombination aus CBD und THC oder eine Isoform, ein Derivat, eine Vorstufe oder einen Metaboliten davon umfasst.

9. Zusammensetzung nach einem der vorhergehenden Ansprüche zur Verwendung bei der Behandlung oder Vorbeugung von Erkrankungen oder Störungen aus der Gruppe bestehend aus immunologischen Erkrankungen, karzinogenen Erkrankungen, endokrinen Störungen, neurologischen Erkrankungen, entzündlichen Erkrankungen, kardiovaskulären Erkrankungen oder Kombinationen davon.

10. Zusammensetzung nach einem der Ansprüche 1 bis 8 oder Zusammensetzung zur Verwendung nach Anspruch 9, wobei die Zusammensetzung in der Form vorliegt, die eine Tablette, eine Kapsel, ein Caplet, eine Pastille, eine Lutschtablette, ein orales Pulver, eine Lösung, einen dünnen Streifen, eine orale dünne Folie (OTF), einen oralen Streifen, einen Sirup, eine Suspension oder eine Inhalationsverbindung umfasst.

11. Cannabinoid-Zusammensetzung, umfassend ein oder mehrere Cannabinoide, vorzugsweise in Form einer natürlichen Schmelztablette, die einen Wirkstoff in Form eines industriellen Cannabisharzes umfasst, das CBD und THC umfasst;
wobei das industrielle Cannabisharz von etwa 10 % (Gew./Gew.) bis etwa 50 % (Gew./Gew.) CBD und von etwa 0,2 % (Gew./Gew.) bis etwa 9,0 % (Gew./Gew.) THC umfasst; wobei die Zusammensetzung besteht aus
- von 10 % (Gew./Gew.) bis 65 % (Gew./Gew.) des industriellen Cannabisharzes,
- von 5 % (Gew./Gew.) bis 25 % (Gew./Gew.) Cannabismehl,
- von 1 % (Gew./Gew.) bis 8 % (Gew./Gew.) Honig, vorzugsweise Akazienhonig und/oder Kastanienhonig, besonders bevorzugt ökologisch,
- von 4 % (Gew./Gew.) bis 20 % (Gew./Gew.) Mohn, vorzugsweise Blaumohn (*Papaver somniferum*),
- von 1 % (Gew./Gew.) bis 5 % (Gew./Gew.) Kurkuma, vorzugsweise Kurkumawurzel,
- von 11 % (Gew./Gew.) bis 53 % (Gew./Gew.) Cannabisblätter, vorzugsweise fein gemahlen, und
- von 1 % (Gew./Gew.) bis 8 % (Gew./Gew.) Cannabisprotein.

12. Zusammensetzung nach einem der vorhergehenden Ansprüche, ferner umfassend 15 % (Gew./Gew.) bis 40,5 % (Gew./Gew.) CBD, vorzugsweise in reiner (99 Gew.-%) kristalliner Form.

13. Schmelztablette, umfassend von 30% (Gew./Gew.) bis 60% (Gew./Gew.) der Zusammensetzung nach einem der Ansprüche 1 bis 12 und mindestens einen pharmazeutisch unbedenklichen Inhaltsstoff als ein Haftmittel, einen Hilfsstoff oder Sprengmittel.

14. Schmelztablette, umfassend,
- von 30,00 mg bis 100,00 mg industrielles Cannabisharz, wobei das industrielle Cannabisharz CBD und/oder THC umfasst, wobei das industrielle Cannabisharz von etwa 10 % (Gew./Gew.) bis etwa 50 % (Gew./Gew.) CBD und von etwa 0,2 % (Gew./Gew.) bis etwa 9,0 % (Gew./Gew.) THC umfasst;
- von 150,00 mg bis 400 mg zusätzliches CBD, vorzugsweise in reiner (99 %) kristalliner Form;
- von 10,00 mg bis 120 mg Cannabismehl;
- von 0,05 mg bis 15 mg Honig, vorzugsweise Akazienhonig und/oder Kastanienhonig, besonders bevorzugt ökologisch,
- von 0,05 mg bis 50,00 mg Mohn, vorzugsweise Blaumohn,
- von 0,05 mg bis 10,00 mg Kurkuma, vorzugsweise Kurkumawurzeln,
- von 1,00 mg bis 120,00 mg Cannabisblätter, vorzugsweise gemahlen,
- von 0,05 mg bis 15,00 mg Cannabisprotein,
und mindestens einen pharmazeutisch unbedenklichen Inhaltsstoff als ein Haftmittel, einen Hilfsstoff oder Sprengmittel.

15. Zusammensetzung nach einem der Ansprüche 1 bis 8 oder 11 bis 12 oder Schmelztablette nach einem der Ansprüche 13 bis 14 zur Verwendung bei der Behandlung oder Vorbeugung von Erkrankungen oder Störungen aus der Gruppe bestehend aus immunologischen Erkrankungen, karzinogenen Erkrankungen, endokrinen Störungen, neurologischen Erkrankungen, entzündlichen Erkrankungen, kardiovaskulären Erkrankungen oder Kombinationen davon.

## Revendications

1. Composition pour administration orale comprenant un ou plusieurs cannabinoïdes, de préférence sous la forme d'un comprimé orodispersible naturel, comprenant un principe actif sous la forme d'une résine de cannabis industrielle comprenant du CBD et du THC ; ladite résine de cannabis industrielle comprenant d'environ 10 % (p/p) à environ 50 % (p/p) de CBD et d'environ 0,2 % (p/p) à environ 9,0 % (p/p) de THC, ladite composition étant composée
- de 10 % (p/p) à 65 % (p/p) de ladite résine de cannabis industrielle,
- de 5 % (p/p) à 25 % (p/p) de farine de cannabis,
- de 1 % (p/p) à 8 % (p/p) de miel, de préférence de miel d'acacia et/ou de miel de châtaignier, plus préférablement écologique,
- de 4 % (p/p) à 20 % (p/p) de pavot, de préférence de pavot bleu (papaver somniferum),
- de 1 % (p/p) à 5 % (p/p) de curcuma, de préférence de racine de curcuma,
- de 11 % (p/p) à 53 % (p/p) de feuilles de cannabis, de préférence finement broyées, et
- de 1 % (p/p) à 8 % (p/p) de protéines de cannabis.

2. Composition selon la revendication 1, ladite composition étant composée
- de 50 % (p/p) à 55 % (p/p) de résine de cannabis industrielle,
- de 9 % (p/p) à 12 % (p/p) de farine de cannabis,
- de 2 % (w/w) à 3 % (w/w)) de miel, de préférence de miel d'acacia et/ou de miel de châtaignier, plus préférablement écologique,
- de 7 % (p/p) à 9 % (p/p) de pavot, de préférence de pavot bleu (papaver somniferum),
- de 1 % (p/p) à 2 % (p/p) de curcuma, de préférence de racine de curcuma,
- de 15 % (p/p) à 29 % (p/p) de feuilles de cannabis, de préférence finement broyées, et
- de 2 % (p/p) à 3 % (p/p) de protéines de cannabis.

3. Composition selon l'une quelconque des revendications précédentes, ladite composition étant composée de
- environ 52,5 % (p/p) de résine de cannabis industrielle,
- environ 10,5 % (p/p) de farine de cannabis,
- environ 2,6 % (p/p) de miel, de préférence de miel d'acacia et/ou de miel de châtaignier, plus préférablement écologique ;
- environ 8,1 % (p/p) de pavot, de préférence de pavot bleu (papaver somniferum),
- environ 1,6 % (p/p) de curcuma, de préférence de racine de curcuma,
- environ 22,1 % (p/p) de feuilles de cannabis, de préférence finement broyées, et
- environ 2,6 % (p/p) de protéines de cannabis.

4. Composition selon l'une quelconque des revendications précédentes, environ 40 % (p/p) à environ 60 % (p/p) de la composition comprenant en outre d'environ 0,8 % (p/p) à environ 3 % (p/p) de vecteur actif de la substance active.

5. Composition selon la revendication 4, ladite substance active étant du dioxyde de silicium colloïdal.

6. Composition selon l'une quelconque des revendications précédentes, ladite composition pour administration orale se présentant sous la forme d'un comprimé orodispersible naturel, ledit comprimé orodispersible naturel étant constitué d'une composition, de CBD et de THC ajoutés, et de cannabinoïdes naturels et de principes actifs naturels ajoutés.

7. Composition selon l'une quelconque des revendications précédentes, ledit comprimé orodispersible naturel pouvant être de n'importe quelle forme comprimée régulière ou irrégulière, et en outre, enrobé d'un enrobage de stéarate de magnésium.

8. Composition selon l'une quelconque des revendications précédentes, ladite composition comprenant une combinaison de CBD et de THC, ou une isoforme, un dérivé, un précurseur ou un métabolite de ceux-ci.

9. Composition selon l'une quelconque des revendications précédentes pour une utilisation dans le traitement ou la prévention de maladies ou de troubles du groupe constitué par les maladies immunologiques, les maladies cancérigènes, les troubles endocriniens, les maladies neurologiques, les maladies inflammatoires, les maladies cardiovasculaires ou une combinaison de ceux-ci.

10. Composition selon l'une quelconque des revendications 1 à 8 ou composition pour utilisation selon la revendication 9, ladite composition se présentant sous la forme comprenant un comprimé, une gélule, un caplet, un trochisque, une pastille, une poudre orale, une solution, une fine bandelette, un film mince oral (OTF), une bandelette orale, un sirop, une suspension ou un composé pour inhalation.

11. Composition cannabinoïde comprenant un ou plusieurs cannabinoïdes, de préférence sous la forme d'un comprimé orodispersible naturel, comprenant un principe actif sous la forme d'une résine de cannabis industrielle comprenant du CBD et du THC ;
ladite résine de cannabis industrielle comprenant d'environ 10 % (p/p) à environ 50 % (p/p) de CBD et d'environ 0,2 % (p/p) à environ 9,0 % (p/p) de THC ;
ladite composition étant composée
- de 10 % (p/p) à 65 % (p/p) de ladite résine de cannabis industrielle,
- de 5 % (p/p) à 25 % (p/p) de farine de cannabis,
- de 1 % (p/p) à 8 % (p/p) de miel, de préférence de miel d'acacia et/ou de miel de châtaignier, plus préférablement écologique,
- de 4 % (p/p) à 20 % (p/p) de pavot, de préférence de pavot bleu (papaver somniferum),
- de 1 % (p/p) à 5 % (p/p) de curcuma, de préférence de racine de curcuma,
- de 11 % (p/p) à 53 % (p/p) de feuilles de cannabis, de préférence finement broyées, et
- de 1 % (p/p) à 8 % (p/p) de protéines de cannabis.

12. Composition selon l'une quelconque des revendications précédentes, comprenant en outre 15 % (p/p) à 40,5 % (p/p) de CBD, de préférence sous une forme cristalline pure (99 % en p/p).

13. Comprimé orodispersible comprenant de 30 % (p/p) à 60 % (p/p) de ladite composition selon l'une quelconque des revendications 1 à 12, et au moins un ingrédient pharmaceutiquement acceptable en tant qu'agent adhésif, excipient ou délitant.

14. Comprimé orodispersible, comprenant,
- de 30,00 mg à 100,00 mg de résine de cannabis industrielle, ladite résine de cannabis industrielle comprenant du CBD et/ou du THC, ladite résine de cannabis industrielle comprenant d'environ 10 % (p/p) à environ 50 % (p/p) de CBD et d'environ 0,2 % (p/p) à environ 9,0 % (p/p) de THC ;
- de 150,00 mg à 400 mg de CBD supplémentaire, de préférence sous une forme cristalline pure (99 %) ;
- de 10,00 mg à 120 mg de farine de cannabis ;
- de 0,05 mg à 15 mg de miel, de préférence du miel d'acacia et/ou du miel de châtaignier, plus préférablement écologique,
- de 0,05 mg à 50,00 mg de pavot, de préférence de pavot bleu,
- de 0,05 mg à 10,00 mg de curcuma, de préférence de racines de curcuma,
- de 1,00 mg à 120,00 mg de feuilles de cannabis, de préférence broyées,
- de 0,05 mg à 15,00 mg de protéines de cannabis,
et au moins un ingrédient pharmaceutiquement acceptable en tant qu'agent adhésif, excipient ou délitant.

15. Composition selon l'une quelconque des revendications 1 à 8 ou 11 à 12, ou comprimé orodispersible selon l'une quelconque des revendications 13 à 14 pour utilisation dans le traitement ou la prévention des maladies ou des troubles du groupe constitué par les maladies immunologiques, les maladies cancérigènes, les troubles endocriniens, les maladies neurologiques, les maladies inflammatoires, les maladies cardiovasculaires, ou une combinaison de ceux-ci.
